# EUROPÄISCHE PATENTSCHRIFT

(11) **EP 2 275 525 B1**
(45) Veröffentlichungstag und Bekanntmachung des Hinweises auf die Patenterteilung: **27.02.2013**
(21) Anmeldenummer: 09009128.1
(22) Anmeldetag: 13.07.2009
(51) Int. Cl.: C12M 1/107, C12M 1/04

(54) **Vorrichtung zur Gewinnung von Biogas**
Device for generating biogas
Dispositif de production de biogaz

(43) Veröffentlichungstag der Anmeldung: 19.01.2011
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A- 1 428 868
- EP-A- 1 736 535
- WO-A-02/06439
- DE-U1-202006 002 757

## Beschreibung

Die Erfindung betrifft eine Vorrichtung zur Gewinnung von Biogas nach dem Oberbegriff des Anspruchs 1.

Bei entsprechenden Biogasanlagen, die insbesondere im Bereich der Trockfermentation eingesetzt werden, wird Biomasse in einem zumeist boxenförmigen Fermenter durch Perkolieren mit einem Perkolat vergoren, so dass Biogas entsteht. Dabei wird das Perkolat in aller Regel aufgefangen und in einen externen Perkolatbehälter befördert, von wo aus wiederum der Fermenter rezyklierend mit Perkolat beschickt wird.

Hierbei ist es wichtig, dass die Temperatur des Substrates bzw. des Perkolates nicht zu stark abfällt, da ansonsten die Vergärung gehemmt wird. Bei den bekannten Anlagen spielen jedoch die auftretenden Wärmeverluste eine wesentliche Rolle.

Man bedient sich zur Lösung des Problems einerseits mit der Heizung des Perkolats im Perkolattank oder auch mit der Heizung des Substrates im Fermenter. Eine Vorrichtung nach dem Oberbegriff des Anspruchs 1 ist aus DE 20 2006 002 757 U1 bekannt. Dort ist ein Fermenter in Form einer Fertiggarage beschrieben. Zur Heizung des Substrates ist in der Sohle des Fermenters eine Fußbodenheizung vorgesehen.

Beide genannten Vorgehensweisen erfordern letztlich aber das Zuführen von Energie, so dass die bisherigen Mittel in puncto Energieeffizienz unbefriedigend erscheinen.

Aufgabe der vorliegenden Erfindung ist es daher, eine Vorrichtung der eingangs genannten Art anzugeben, bei der diese Nachteile reduziert auftreten und die hinsichtlich der Energiebilanz wirtschaftlicher arbeitet.

Gelöst wird diese Aufgabe mit den Merkmalen des Anspruchs 1. Vorteilhafte Ausführungsformen finden sich in den abhängigen Ansprüchen.

Erfindungsgemäß ist wenigstens jenseits der Bodenfläche wenigstens eines Fermenters eine Perkolatsammeleinrichtung und ein Wärmereservoir vorgesehen. Bevorzugt dient der Perkolatbehälter als Wärmereservoir. Durch die fermenternahe Anordnung der Perkolatsammeleinrichtung und des Wärmereservoirs, insbesondere unterhalb des Fermenters, können insbesondere Rohrleitungen zur Verbindung von Sandfang, Fermenter und Perkolatbehälter praktisch ersatzlos entfallen, so dass eine in der Vergangenheit wesentliche Quelle von Wärmeverlusten beseitigt wird. Zudem können Perkolatsammeleinrichtung und insbesondere der Perkolatbehälter selbst als Wärmereservoir betrieben werden. Das Perkolat, welches aus dem Fermenter im Prozess in den Perkolatbehälter gelangt, dient als Wärmequelle und als Heizung für das im Fermenter befindliche Substrat. Im Perkolatbehälter kann vorteilhafterweise eine Heizung zur Erwärmung des Perkolates vorgesehen werden.

Bevorzugt ist die Perkolatsammeleinrichtung als Sandfang und/oder Sammelleitung für Perkolatströme ausgebildet.

Nach einer besonders bevorzugten Ausführungsform ist die Anlage modular aufgebaut, wobei jedes Modul dabei aus einem Fermenter, einem Sandfangabschnitt und/oder einem Perkolatbehälterabschnitt aufgebaut ist. Bevorzugt sind die Module boxenförmig aufgebaut, so dass sie nebeneinander aufgestellt werden können und sich dabei die Sandfangabschnitte und/oder Perkolatbehälterabschnitte zu einem Sandfang bzw. Perkolatbehälter zusammensetzen. Der Perkolatbehälter hat dabei einen Einlass und einen Auslass und weist zwischen Ein- und Auslass bevorzugt Wandabschnitte auf, die so angeordnet sind, dass das Perkolat im Perkolatbehälter zwischen Ein- und Auslass mäanderartig geführt wird und so eine ständige Durchmischung des Perkolats ermöglicht.

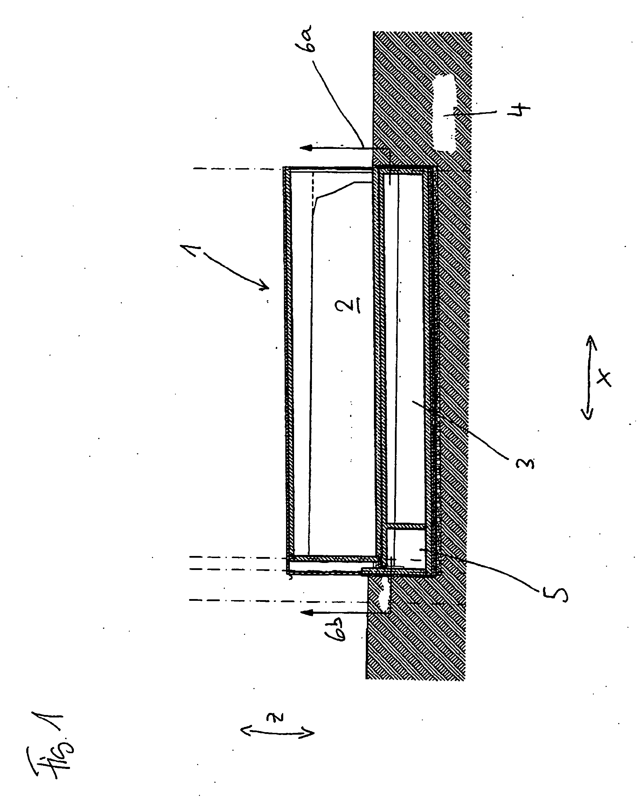

Die Erfindung wird nun anhand der Ausführungsbeispiele in den Figuren 1 bis 3B schematisch näher erläutert. Im Folgenden wird lediglich beispielhaft von Sandfang gesprochen. Dies ist lediglich eine spezielle Ausgestaltung der Perkolatsammeleinrichtung. Alternativ gilt die folgende Beschreibung auch für eine Sammelleitung oder eine andere Feststoffabscheideeinrichtung oder dergleichen.
- Figur 1 -: zeigt eine Schnittansicht durch eine erfin- dungsgemäße Vorrichtung,
- Figur 2 -: zeigt eine Ausschnittvergrößerung der Figur 1,
- Figur 3A -: zeigt den Grundriss der ersten Etage einer erfindungsgemäßen Vorrichtung mit mehreren Fermentern,
- Figur 3B -: zeigt eine unter der ersten Etage liegende Etage einer erfindungsgemäßen Vorrichtung mit mehreren Fermentern.

Die Schnittdarstellung der Figur 1 zeigt einen Fermenter 1, der hier als Boxenfermenter ausgebildet ist und sich auf Bodenniveau befindet, um etwa mit einem Radlader mit zu vergärendem Substrat 2 beschickt zu werden. Der Fermenter 1 weist einen Boden 1a auf, auf welchem das Substrat 2 liegt. Unterhalb des Bodenabschnitts 1a ist zum einen ein Sandfang 5 im Bereich eines stirnseitigen Endes des Fermenters 1 vorgesehen. An den Sandfang grenzt unterhalb des Bodenabschnitts 1a des Fermenters 1 ein Perkolatbehälter 3 an. Sowohl Sandfang 5 als auch Perkolatbehälter 3 haben Biogasauslässe, um dort entstehendes Biogas über Leitungen 6b, 6a abzuführen. Entsprechende Auslässe weist auch der Fermenter 1 auf (hier nicht gezeigt). Sandfang 5 und Perkolatbehälter 3 sind wenigstens teilweise in das Erdreich 4 eingebettet, so dass dort entstehende Abwärme nicht vorrangig an die Atmosphäre abgegeben werden kann. Die Bodenfläche 1a des Fermenters 1 ist geneigt ausgeführt, im gezeigten Beispiel fällt die Bodenfläche 1a, auf der das Substrat 2 liegt, zu dem Ende des Fermenters 1 hin ab, in dessen Bereich der Sandfang 5 angeordnet ist. So fließt das durch das Substrat 2 im Prozess sickernde Perkolat am Boden 1a des Fermenters 1 zum genannten Ende hin und kann dort in den Sandfang 5 geführt werden.

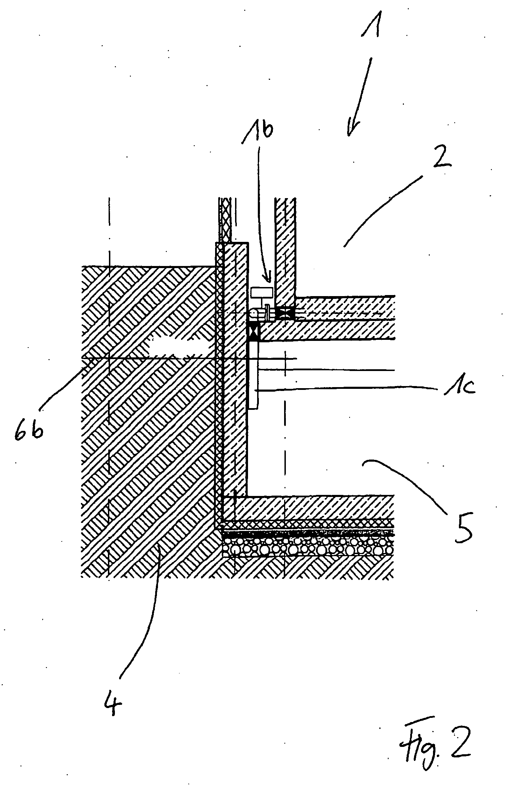

Wie in Figur 2 gezeigt, ist an diesem Ende des Fermenters 1 im Boden 1a ein absperrbarer Auslass 1b vorgesehen. Dieser Auslass führt über eine kurze Rohrleitung 1c in den Sandfang 5, und zwar so, dass kein Gas über die Leitung 1c ausgetauscht werden kann. Die ansonsten einige Meter lange Leitung 1c zwischen Fermenter 1 und einem Sandfang 5 wird durch die erfindungsgemäße Anordnung des Sandfangs 5 auf einige Zentimeter reduziert.

Des weiteren ist zwischen Sandfang 5 und Perkolatbehälter 3 eine absperrbare Verbindung (hier nicht gezeigt) vorgesehen, durch welche das im Sandfang 5 gereinigte Perkolat in den Perkolatbehälter 3 gelangt und von dort aus wieder in den Fermenter 1 rezykliert werden kann. Wie in Figur 1 gezeigt, erstreckt sich der in das Erdreich 4 eingebettete Perkolatbehälter 3 über einen wesentlichen Teil der Länge des Fermenters 1, so dass eine große Wärmeaustauschfläche zwischen Fermenter 1 und Perkolatbehälter 3 zur Verfügung steht. Der Perkolatbehälter und in gewissen Grenzen auch der flächenmäßig wesentlich kleinere Sandfang 5 können erfindungsgemäß als Wärmereservoir zur Erwärmung des Substrates 2 im Fermenter betrieben und genutzt werden. Durch die Erwärmung des Perkolats im Perkolatbehälter 3 kann so auch das Substrat 2 über die zur Verfügung gestellte Wärmetauschfläche beheizt werden. Rohrleitungen zum Transport des Perkolats sind kaum noch erforderlich.

Die Erfindung ermöglicht zudem eine im Vergleich zu konventionellen Anlagen Platz sparendere Ausführung. Dies wird zum Einen durch die Etagenbauweise ermöglicht, zum Anderen kann der Aufbau modular erfolgen, wobei der Perkolatbehälter entsprechend den Anforderungen bei wachsenden Anlagen "mitwächst".

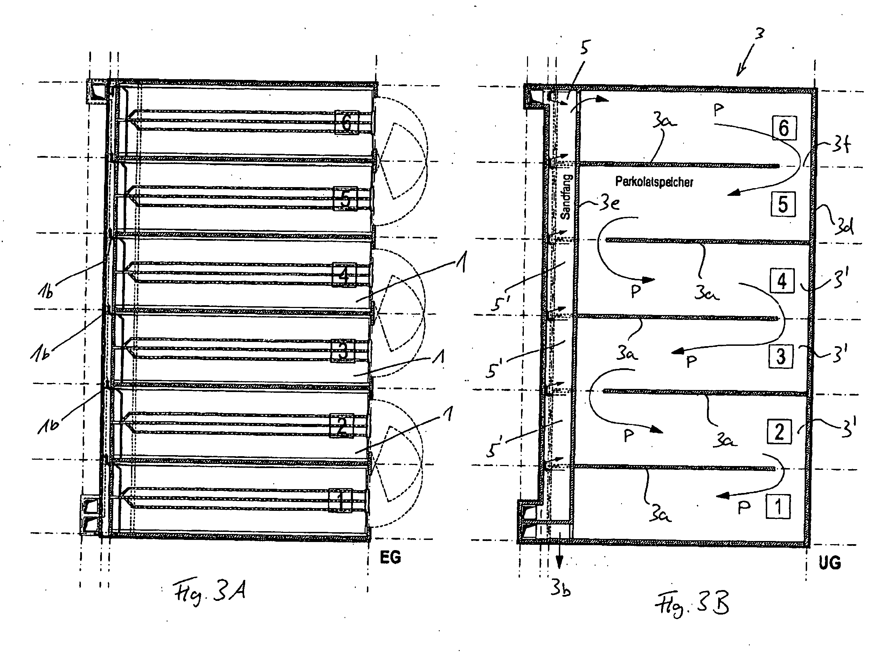

In den Figuren 3A und 3B ist eine solche Anlage im Grundriss durch die erste Etage aus Fermentern 1 (Erdgeschoss, EG) und die darunterliegende Etage aus Sandfang 5 und Perkolatbehälter 3 (Untergeschoss, UG) dargestellt, welche im gezeigten Beispiel aus 6 Modulen zusammengesetzt ist. Im Längsschnitt entspricht das EG im Wesentlichen der in Figur 1 dargestellten Schnittdarstellung. Jeder Fermenter 1 weist einen Auslass 1b auf, durch welchen das aufgefangene Perkolat in den Sandfang 5 geführt wird, so dass unterhalb jedes Fermenters 1 Perkolat in einen zugehörigen Sandfangabschnitt 5' gelangt. An jeden der Sandfangabschnitte 5', die zusammengenommen den Sandfang 5 bilden, an dessen Ende eine verschließbare Öffnung 5a vorgesehen ist, grenzt ein Perkolatbehälterabschnitt 3' an. Ein Modul wird also durch einen Fermenter 1 und einen darunter befindlichen Sandfangabschnitt 5' und einen Perkolatbehälterabschnitt 3' gebildet. Die Perkolatbehälterabschnitte 3' bilden wiederum den Perkolatbehälter 3, der seinerseits einen Einlass 3c zur Verbindung mit dem Auslass 5a des Sandfangs 5 und einen Auslass 3b zur Entnahme des Perkolats aufweist, welches dann ggf. wieder in einen Fermenter 1 geleitet werden kann.

Bevorzugt ist nun vorgesehen, dass lediglich ein Einlass 3c und der Auslass 3b für den modular aufgebauten Perkolatbehälter 3 vorgesehen sind. Bevorzugt ist weiter vorgesehen, dass der Strömungsweg zwischen Ein- und Auslass im Perkolatbehälter möglichst lang ist. Dazu sind im Perkolatbehälter 3 Zwischenwände gezogen, die im Wesentlichen in vertikaler Richtung Z mit den Längsseiten der Fermenter 1 fluchten. Die Zwischenwände 3a erstrecken sich bevorzugt in Längserstreckung X der Fermenter, quer zur Richtung Y der nebeneinander angeordneten Module bzw. quer zur Längsrichtung des modular aufgebauten Sandfangs 5. Die Zwischenwände erstrecken sich dabei jedoch nicht über die volle Länge zwischen zwei Stirnseiten 3e, 3d der Perkolatbehälterabschnitte 3', sondern lassen zu jeweils einer Stirnseite 3e, 3d einen Abstand 3f offen, so dass das Perkolat - wie durch die Pfeile P angedeutet - mäanderförmig zwischen Einlass 3c und Auslass 3b durch den Perkolatbehälter 3 fließen kann. Dadurch wird eine vergleichmäßigte Aufenthaltszeit des Perkolats erreicht, so dass insbesondere eine homogene Temperaturverteilung im Perkolat ermöglicht wird.

Mit der vorliegenden Erfindung kann zum Einen der Perkolatbehälter synergetisch zur Temperierung des Substrats 2 eingesetzt werden; des Weiteren ergeben sich aufgrund dieser Bauweise Synergieeffekte, die zu einer kompakteren und energiesparenderen Konzipierung von entsprechenden Fermentationsanlagen und zur Modularisierbarkeit solcher Anlagen führen.

## Patentansprüche

1. Vorrichtung zur Gewinnung von Biogas mit wenigstens einem Fermenter (1) zur Aufnahme eines zu perkolierenden Substrates (2) sowie einem Perkolatbehälter (3), wobei Fermenter (1) und Perkolatbehälter (3) miteinander so verschaltet sind, dass das im Fermenter (1) durch das Substrat (2) sickernde Perkolat in den Perkolatbehälter (3) gelangt und von dort aus gegebenenfalls wieder in den Fermenter (1) zurückgeführt werden kann, wobei wenigstens teilweise jenseits einer Bodenfläche (1a) des Fermenters (1) ein Wärmereservoir (3) und eine Perkolatsammeleinrichtung (5) angeordnet sind,
**dadurch gekennzeichnet,**
**dass** die Perkolatsammeleinrichtung (5) zwischen Fermenter (1) und Perkolatbehälter (3) geschaltet und unterhalb der Bodenfläche (1a) des Fermenters (1) angeordnet ist.

2. Vorrichtung nach Anspruch 1,
**dadurch gekennzeichnet,**
**dass** das Wärmereservoir durch den Perkolatbehälter (3) gebildet wird.

3. Vorrichtung nach Anspruch 1 oder 2,
**dadurch gekennzeichnet,**
**dass** der Perkolatbehälter (3) unterhalb des Fermenters (1) wenigstens teilweise im Erdreich (4) vorgesehen ist.

4. Vorrichtung nach einem der vorigen Ansprüche,
**dadurch gekennzeichnet,**
**dass** die Perkolatsammeleinrichtung (5) angrenzend an den Perkolatbehälter (3) vorgesehen ist.

5. Vorrichtung nach einem der vorherigen Ansprüche,
**dadurch gekennzeichnet,**
**dass** der Fermenter (1) eine Bodenfläche (1a) aufweist, welche wenigstens abschnittweise geneigt ausgebildet ist, so dass durch das Substrat sickerndes Perkolat in Richtung einer Wand und/oder Ecke des Fermenters fließen kann.

6. Vorrichtung nach einem der vorherigen Ansprüche,
**dadurch gekennzeichnet,**
**dass** der Fermenter (1) wenigstens einen Auslass (1b) zum Auslassen des Perkolats aufweist.

7. Vorrichtung nach einem der vorherigen Ansprüche,
**dadurch gekennzeichnet,**
**dass** eine Mehrzahl Fermenter (1) vorgesehen sind, welche nebeneinander angeordnet sind und unterhalb derer ein gemeinsamer Perkolatbehälter (3) vorgesehen ist.

8. Vorrichtung nach Anspruch 7,
**dadurch gekennzeichnet,**
**dass** im Perkolatbehälter (3) Zwischenwände (3a) vorgesehen sind, die so angeordnet sind, dass in den Perkolatbehälter (3) gelangendes Perkolat zwischen einem Perkolateinlass (3c) und einem Perkolatauslass (3b) mäandrierend durch den Perkolatbehälter (3) geführt wird.

9. Vorrichtung nach Anspruch 7 oder 8,
**dadurch gekennzeichnet,**
**dass** die Auslässe (1b) der Fermenter (1) in einer gemeinsamen Perkolatsammeleinrichtung (5) münden, welcher sich entlang der Fermenter (1) erstreckt und einen Sammelauslass (5a) aufweist, welcher in den Perkolateinlass (3c) des Perkolatbehälters (3) mündet.

10. Vorrichtung nach Anspruch 9,
**dadurch gekennzeichnet, dass** diese eine Mehrzahl Module aufweist, wobei wenigstens zwei Module einen Fermenter (1), einen Perkolatsammeleinrichtungsabschnitt (5') und einen Perkolatbehälterabschnitt (3') aufweist, wobei jeder Fermenter (1) einen in den zugehörigen Perkolatsammeleinrichtungsabschnitt (5') mündenden Auslass (1a) aufweist.

11. Vorrichtung nach einem der vorherigen Ansprüche,
**dadurch gekennzeichnet,**
**dass** die Perkolatsammeleinrichtung (5) als Sammelleitung oder/und als Sandfang ausgebildet ist.

## Claims

1. Device for obtaining biogas having at least one fermenter (1) for receiving a substrate (2) which is to be percolated, as well as a percolate container (3), wherein the fermenter (1) and percolate container (3) are connected to one another so that the percolate seeping through the substrate (2) in the fermenter (1) passes into the percolate container (3) and can be returned from there when necessary back again into the fermenter (1), wherein at least in part on the other side of a base surface (1a) of the fermenter (1) there is a heat reservoir (3) and a percolate collecting device (5),
**characterised in that**
the percolate collecting device (5) is connected in between the fermenter (1) and the percolate container (3) and is arranged underneath the base surface (1a) of the fermenter (1).

2. Device according to claim 1,
**characterised in that**
the heat reservoir is formed by the percolate container (3).

3. Device according to claim 1 or 2,
**characterised in that**
the percolate container (3) is provided underneath the fermenter (1) at least partially in the ground d (4).

4. Device according to one of the preceding claims,
**characterised in that**,
the percolate collecting device (5) is provided adjoining the percolate container (3).

5. Device according to one of the preceding claims,
**characterised in that**,
the fermenter (1) has a base surface (1a) which is formed inclined in at least some sections so that percolate seeping through the substrate can flow in the direction of a wall and/or corner of the fermenter.

6. Device according to one of the preceding claims
**characterised in that**,
the fermenter (1) has at least one outlet (1b) for letting out the percolate.

7. Device according to one of the preceding claims,
**characterised in that**
a number of fermenters (1) are provided which are arranged side by side and a common percolate container (3) is provided underneath same.

8. Device according to claim 7,
**characterised in that**
partitions (3a) are provided in the percolate container (3) and are arranged so that percolate passing into the percolate container (3) is guided meandering through the percolate container (3) between a percolate inlet (3c) and a percolate outlet (3b).

9. Device according to claim 7 or 8,
**characterised in that**
the outlets (1b) of the fermenter (1) open in a common percolate collecting device (5) which extends along the fermenter (1) and has a collecting outlet (5a) which opens into the percolate inlet (3c) of the percolate container (3).

10. Device according to claim 9,
**characterised in that**
this has a number of modules wherein at least two modules have a fermenter (1), a percolate collecting device section (5') and a percolate container section (3') wherein each fermenter (1) has an outlet (1a) opening into the associated percolate collecting device section (5').

11. Device according to one of the preceding claims,
**characterised in that**
the percolate collecting device (5) is designed as a collecting pipe or/and as a sand trap.

## Revendications

1. Dispositif pour la production de biogaz, avec au moins un fermenteur (1) pour la réception d'un substrat de percolation, ainsi qu'un réservoir de percolat (3), le fermenteur (1) et le réservoir de percolat (3) étant connectés ensemble de sorte que le percolat, qui s'écoule goutte à goutte dans le fermenteur (1), à travers le substrat (2), parvienne dans le réservoir de percolat (3) et puisse, de là, être reconduit dans le fermenteur (1), sachant qu'un réservoir de chaleur (3) et un collecteur de percolat (5) sont disposés, au moins partiellement, au-delà d'une surface de fond (1a) du fermenteur (1),
**caractérisé en ce que**
le collecteur de percolat (5) est connecté entre le fermenteur (1) et le réservoir de percolat (3) et est disposé au-dessous de la surface de fond (1a) du fermenteur (1).

2. Dispositif selon la revendication 1,
**caractérisé en ce que**
le réservoir de chaleur est formé par le réservoir de percolat (3).

3. Dispositif selon revendication 1 ou 2,
**caractérisé en ce que**
le réservoir de percolat (3) est prévu au-dessous du fermenteur (1), au moins partiellement dans la terre (4).

4. Dispositif selon l'une des revendications précédentes,
**caractérisé en ce que**
le collecteur de percolat (5) est adjacent au réservoir de percolat (3).

5. Dispositif selon l'une des revendications précédentes,
**caractérisé en ce que**
le fermenteur (1) présente une surface de fond (1a), qui est inclinée, au moins par sections, de sorte que le percolat, qui s'écoule goutte à goutte à travers le substrat, puisse couler en direction d'une paroi et / ou d'un angle du fermenteur.

6. Dispositif selon l'une des revendications précédentes,
**caractérisé en ce que**
le fermenteur (1) présente au moins une sortie (1b) pour l'évacuation du percolat.

7. Dispositif selon l'une des revendications précédentes,
**caractérisé en ce que**
sont prévus plusieurs fermenteurs (1), qui sont disposés les uns à côté des autres et sous lesquels est prévu un réservoir de percolat (3) commun.

8. Dispositif selon la revendication 7,
**caractérisé en ce que**,
dans le réservoir de percolat (3), sont prévues des parois intermédiaires (3a), qui sont disposées de sorte que le percolat, parvenu dans le réservoir de percolat (3), soit conduit en méandres, à travers le réservoir de percolat (3), entre une entrée de percolat (3c) et une sortie de percolat (3b)

9. Dispositif selon revendication 7 ou 8,
**caractérisé en ce que**
les sorties (1b) du fermenteur (1) débouchent dans un collecteur de percolat (5) commun, qui s'étend le long du fermenteur (1) et présente une sortie (5a), qui débouche dans l'entrée de percolat (3c) du réservoir de percolat (3).

10. Dispositif selon la revendication 9,
**caractérisé en ce que**
celui-ci présente une pluralité de modules, sachant qu'au moins deux modules présentent un fermenteur (1), une section de collecteur de percolat (5') et une section de réservoir de percolat (3') sachant que chaque fermenteur (1) est doté d'une sortie (1a), qui débouche dans la section de collecteur de percolat (5') y associée.

11. Dispositif selon l'une des revendications précédentes,
**caractérisé en ce que**
le collecteur de percolat (5) est réalisé en tant que conduit collecteur ou / et de dessableur.
